# FASCICULE DE BREVET EUROPEEN

(11) **EP 4 284 237 B1**
(45) Date de publication et mention de la délivrance du brevet: **08.04.2026**
(21) Numéro de dépôt: 22706341.9
(22) Date de dépôt: 25.01.2022
(51) Int. Cl.: A61B 5/02, A61B 5/01

(54) **SYSTÈME DE PRÉDICTION DE RUPTURE OU DE DÉCOLLEMENT DE PLAQUE VASCULAIRE POUVANT ENTRAÎNER UN ACCIDENT VASCULAIRE CÉRÉBRAL ET/OU DE PRÉDICTION D'UNE THROMBOSE VASCULAIRE**
SYSTEM ZUR VORHERSAGE VON GEFÄSSPLAQUERUPTUR ODER -ABLÖSUNG, DIE ZU EINEM SCHLAGANFALL FÜHREN UND/ODER ZUR VORHERSAGE VON GEFÄSSTHROMBOSE FÄHIG SIND
SYSTEM FOR PREDICTING VASCULAR PLAQUE RUPTURE OR DETACHMENT THAT COULD LEAD TO A STROKE AND/OR FOR PREDICTING VASCULAR THROMBOSIS

(30) Priorité: 27.01.2021 FR 2100754; 11.08.2021 FR 2108631
(43) Date de publication de la demande: 06.12.2023
(73) Titulaire: Octogone Medical, 75116 Paris (FR); Université Paris Cité, 75006 Paris (FR); Institut National de la Santé et de la Recherche Médicale, 75013 Paris (FR); Assistance Publique Hôpitaux de Paris, 75012 Paris (FR)
(72) Inventeur: MESSAS, Emmanuel, 75016 Paris (FR); SALDMANN, Frédéric, 75007 Paris (FR)
(74) Mandataire: Plasseraud IP
(86) Numéro de dépôt international: PCT/FR2022/050130
(87) Numéro de publication internationale: WO 2022/162301

(56) Documents cités:
- US-A1- 2003 125 637
- US-A1- 2003 220 556
- US-A1- 2004 102 722
- US-A1- 2012 065 514
- US-A1- 2017 340 393
- US-A1- 2020 015 758
- US-A1- 2020 279 656
- KIPS ET AL: "Identifying the vulnerable plaque: A review of invasive and non-invasive imaging modalities", 20080125, vol. 2, no. 1, 25 January 2008 (2008-01-25), pages 21 - 34, XP022513004

## Description

### Domaine technique

La présente divulgation concerne un système de prédiction de la rupture ou décollement de plaque vasculaire au niveau de la carotide ou des troncs supra-aortiques pouvant entraîner un accident vasculaire cérébral.

La présente divulgation concerne plus particulièrement un système et procédé permettant d'assurer une surveillance continue de l'évolution de la stabilité des plaques vasculaires présentes dans la carotide ou des troncs supra-aortiques d'un patient et de prédire la survenue d'un accident vasculaire cérébral par rupture de plaque vasculaire. Le système est également adapté pour assurer une surveillance continue des propriétés biomécaniques de la veine jugulaire interne qui croise la bifurcation carotidienne pour prédire la survenue d'une thrombose vasculaire.

### Technique antérieure

Un accident vasculaire cérébral (AVC) est lié à un arrêt brutal de la circulation sanguine vers tout ou partie du cerveau.

Le cerveau est irrigué par la carotide interne et par l'ensemble des troncs supra-aortiques en apportant l'oxygène nécessaire à son fonctionnement. Un processus connu sous le nom d'athérome provoque un épaississement et un durcissement des artères par l'accumulation au niveau de la paroi interne des artères de lipides, de glucides complexes, de sang et de dépôts calcaires, et entraîne la formation progressive d'une ou plusieurs plaques d'athérome. Il existe aussi d'autres causes de formation de plaque vasculaire. C'est un processus dont l'évolution est accélérée par des facteurs de risques identifiés, à savoir l'hypertension artérielle, le diabète, l'obésité, le tabagisme. La présence des plaques d'athérome provoque ainsi une réduction du diamètre de l'artère que l'on appelle « sténose », une maladie appelée plus généralement athérosclérose. Cette plaque peut aussi être d'origine post radique ou post inflammatoire ou due à une maladie du collagène. Dans tous ces cas le risque de rupture existe. La présence de la plaque vasculaire (due à l'athérome ou à d'autres origines) peut être détectée par divers techniques, l'échographie Doppler vasculaire, la tomographie par cohérence optique, la tomodensitométrie ou l'Imagerie par Résonance Magnétique.

Une conséquence dramatique liée à la rupture de la plaque au niveau de la carotide ou des troncs supra-aortiques se traduit par la migration de certains composants de la plaque dans la circulation sanguine vers le cerveau, obstruant ainsi une des artères cérébrales, provoquant un AVC ischémique. Un des autres risques évolutifs possibles de la plaque d'athérome qui est plus rare est une rupture de la plaque qui se traduit par l'occlusion de la carotide interne, provoquant un AVC hémodynamique par bas débit en cas de non-compensation du polygone de Willis. Dans chacun des cas, les conséquences dramatiques sont en corrélation avec le décollement ou la rupture d'une partie ou de la totalité de la plaque. La rupture d'une partie de la plaque ou de la plaque entière est suivie le plus souvent par un embole fibrino-cruorique plus ou moins calcifié pouvant obstruer l'artère cérébrale.

L'évaluation actuelle se basant uniquement sur le degré de sténose est loin d'être suffisant et ne permet pas de prédire la rupture de plaque et le risque réel d'AVC. Il est ainsi primordial d'évaluer le risque de rupture de la plaque d'athérome ou d'autres origines afin de définir précisément le risque individuel de survenue d'un AVC.

Actuellement, l'évaluation du risque de rupture des plaques vasculaires est principalement basée sur la morphologie et sur la composition tissulaire de la plaque. En effet, les paramètres morphologiques tels que l'épaisseur de la chape fibreuse, la taille du corps nécrotique et la sténose (l'encombrement de la lumière de l'artère) sont considérés comme des facteurs influençant la survenue de la rupture de la plaque. Les plaques ayant rompu sont caractérisées par un cœur lipidique important et une chape fibreuse fine et une hémorragie intraplaque. Cependant, ces indicateurs ne permettent pas de prédire avec fiabilité le risque de rupture de la plaque vasculaire.

US2017/340393-A1, US2020/279656-A1, et US2020/015758-A1 montrent des systèmes de prédiction d'une rupture de plaque vasculaire utilisant une intelligence artificielle entraînée. US2003/125637-A1, US2004/102722-A1, KIPS ET AL: "Identifying the vulnerable plaque: A review of invasive and non-invasive imaging modalities", vol. 2, no. 1, 25 janvier 2008 pages 21-34, ainsi que US2003/220556-A1, et US2012/065514-A1 montrent d'autres systèmes de prédiction d'une rupture de plaque vasculaire.

Un but de la présente divulgation est de proposer un système qui permet de surveiller en continu l'évolution de l'état de l'intégrité de la plaque vasculaire afin de pouvoir prédire la survenue d'un AVC entraîné par la rupture ou le décollement de plaque.

Un autre but de la présente divulgation est de proposer un système et procédé de prédiction de la survenue d'un AVC qui soit peu contraignante pour le patient et d'une utilisation aisée pour le praticien.

Un autre but de la présente divulgation est de proposer un système et procédé qui permettent de surveiller en continu l'évolution des propriétés biomécaniques d'une veine jugulaire interne afin de prédire le risque d'une thrombose vasculaire.

### Résumé

La présente divulgation vient améliorer la situation.

Il est proposé un système de prédiction d'une rupture au moins partielle ou un décollement de plaque vasculaire pouvant entraîner un accident vasculaire cérébral, ladite plaque vasculaire étant présente sur une paroi artérielle choisie parmi une paroi de carotide et une paroi de tronc supra-aortique, ledit système comprenant :
- un dispositif de surveillance apte à être placé à proximité de la plaque vasculaire, ledit dispositif comprenant au moins un capteur de température configuré pour mesurer la température de la plaque vasculaire, au moins un capteur de vibrations configuré pour mesurer des ondes mécaniques propagées dans ladite paroi artérielle, au moins un capteur de mouvement de la plaque vasculaire configuré pour mesurer des mouvements de la plaque vasculaire, une mémoire apte à stocker des signaux transmis par lesdits capteurs, une interface de communication, une source d'énergie configurée pour alimenter lesdits capteurs et l'interface de communication,
- une unité de calcul adaptée pour communiquer avec le dispositif de surveillance et configurée pour analyser des mesures du capteur de vibrations, du capteur de température et du capteur de mouvement de plaque vasculaire provenant du dispositif de surveillance par une intelligence artificielle entraînée pour détecter s'il y a un risque de rupture au moins partielle ou décollement de la plaque vasculaire.

Selon un mode de réalisation, le dispositif de surveillance comprend en outre au moins un capteur électrique configuré pour mesurer un paramètre lié à l'impédance électrique de la plaque vasculaire.

Selon un autre mode de réalisation, le dispositif de surveillance comprend en outre au moins un capteur d'ondes acoustiques configuré pour mesurer des ondes acoustiques provenant de la paroi artérielle et/ou de la plaque vasculaire.

Grâce au dispositif de surveillance fixé directement sur le patient, il est possible de surveiller en temps réel l'évolution de l'intégrité des plaques vasculaires.

Le système permet de prédire le risque de la survenue d'un AVC sans nécessiter des examens complexes et souvent invasifs du patient.

Les caractéristiques exposées dans les paragraphes suivants peuvent, optionnellement, être mises en oeuvre, indépendamment les unes des autres ou en combinaison les unes avec les autres :

Le dispositif de surveillance se présente sous la forme d'un patch apte à être collé sur une surface externe de la peau d'un patient.

Le dispositif de surveillance se présente sous la forme d'un implant sous-cutanée apte à être inséré sous la peau d'un patient.

Le capteur de température est un capteur d'ondes thermiques émises par la plaque vasculaire.

Le capteur de vibrations comprend un accéléromètre.

Le capteur de vibrations comprend un accéléromètre 3 axes et un gyroscope 3 axes.

Le capteur de mouvement de la plaque vasculaire est une sonde ultrasonore.

La source d'énergie est une pile rechargeable par induction.

L'interface de communication est choisie parmi une interface radio courte portée ou une interface de communication en champ proche.

Selon un autre mode de réalisation, le système comprend en outre au moins un appareil mobile de communication adapté pour communiquer à distance avec le dispositif de surveillance via ladite interface de communication qui est une interface de courte portée et pour transmettre des signaux vers l'unité de calcul via une interface de communication de longue portée appartenant à l'unité de calcul.

Selon un mode de réalisation, le dispositif de surveillance comprend au moins un capteur de température et/ou au moins un capteur de mouvement et/ou au moins un capteur d'ondes acoustiques, la fréquence de mesures desdits capteurs étant réglée pour émettre et/ou recevoir un signal provenant d'une veine jugulaire interne.

Selon un autre aspect, il est proposé un procédé pour prédire une rupture au moins partielle ou un décollement de plaque vasculaire pouvant entraîner un accident vasculaire cérébral, ladite plaque vasculaire étant présente sur une paroi artérielle choisie parmi une paroi de carotide et une paroi de tronc supra-aortique d'un patient en utilisant un système tel que décrit ci-dessus, le procédé comprenant :
- acquérir en continu pendant une durée déterminée une pluralité de signaux représentatifs de l'évolution du risque de rupture ou de décollement de la plaque vasculaire au moyen du dispositif de surveillance placé à proximité de la plaque vasculaire,
- analyser la pluralité de signaux dans une unité de calcul par une intelligence artificielle entraînée pour détecter le risque d'une rupture au moins partielle ou décollement la plaque vasculaire.

Selon un mode de réalisation, l'intelligence artificielle est un réseau neuronal et le procédé comprend en outre une étape d'apprentissage préalable comprenant :
- acquérir une pluralité de signaux, dites signaux de référence provenant des dispositifs de surveillance portés par une population dont les risques de rupture ou décollement de la plaque vasculaire sont connus,
- entraîner le réseau neuronal avec lesdits signaux de référence jusqu'à ce qu'il converge.

### Brève description des dessins

D'autres caractéristiques, détails et avantages apparaîtront à la lecture de la description détaillée ci-après, et à l'analyse des dessins annexés, sur lesquels :
**Fig. 1**

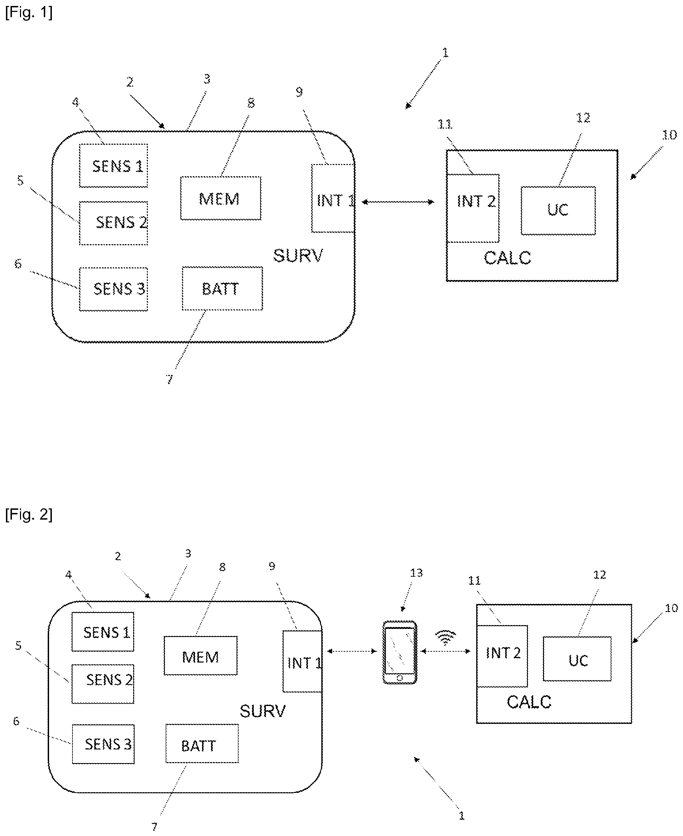

   [Fig. 1] La figure 1 est une illustration d'un système de prédiction d'une rupture ou d'un décollement de plaque vasculaire pouvant entraîner un AVC selon un mode de réalisation.
**Fig. 2**
   [Fig. 2] La figure 2 est une illustration d'un système de prédiction selon un autre mode de réalisation.
**Fig. 3**

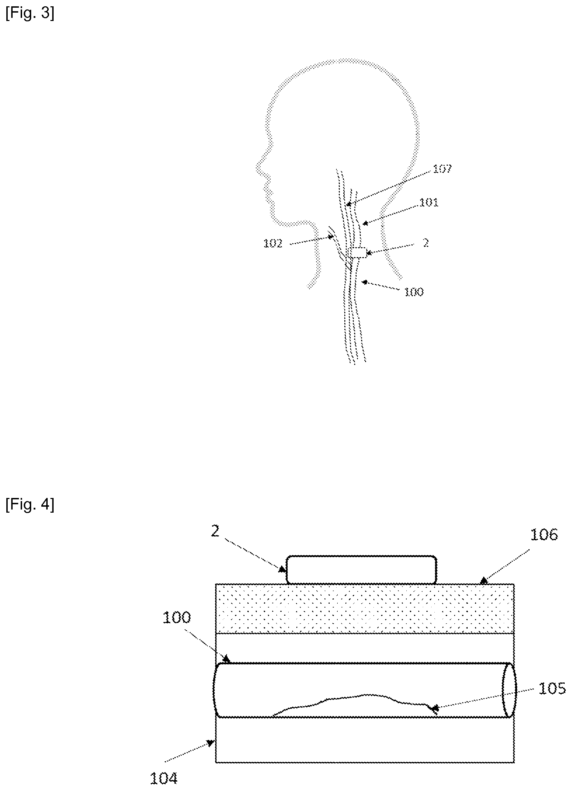

   [Fig. 3] La figure 3 montre le dispositif de surveillance du système de prédiction positionné au niveau du cou d'un patient, à proximité de la carotide interne après la bifurcation de la carotide commune, en regard de plaque vasculaire.
**Fig. 4**
   [Fig. 4] La figure 4 montre un exemple de réalisation du dispositif de surveillance fixé sur une surface externe de la peau, à proximité de la carotide.
**Fig. 5**

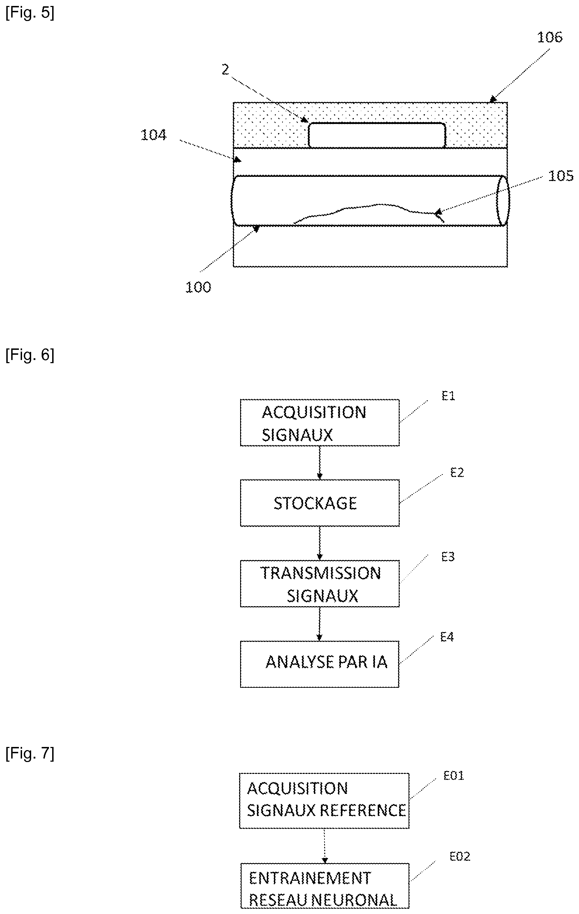

   [Fig. 5] La figure 5 montre un autre exemple de réalisation du dispositif de surveillance implanté sous la peau, à proximité de la carotide.
**Fig. 6**
   [Fig. 6] La figure 6 est un organigramme représentant le procédé de prédiction mis en oeuvre par le système de prédiction selon un mode de réalisation.
**Fig. 7**
   [Fig. 7] La figure 7 est un organigramme représentant l'étape d'apprentissage pour entraîner le réseau neuronal utilisé par l'unité de calcul.
**Fig. 8**

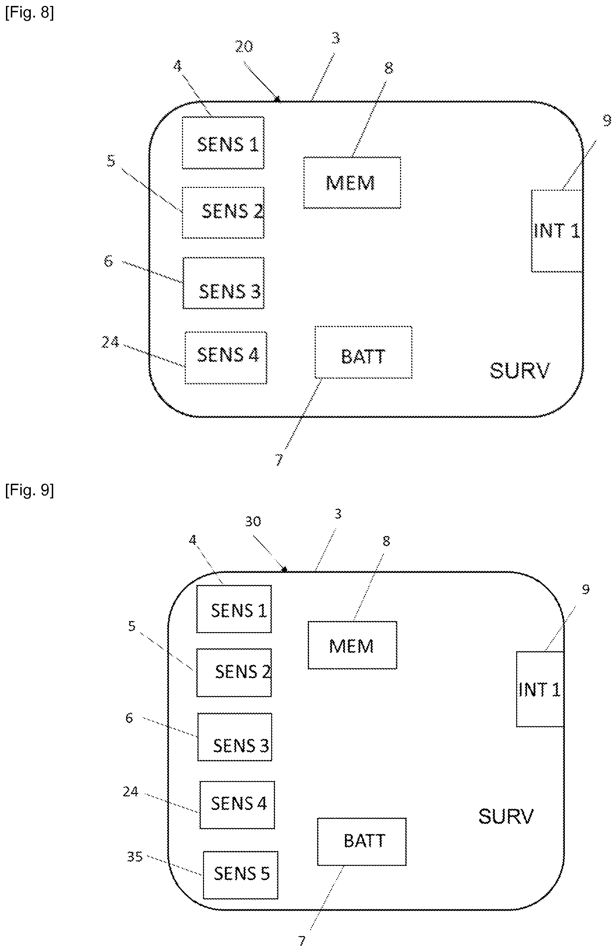

   [Fig. 8] La figure 8 est une illustration schématique du dispositif de surveillance comprenant en outre un capteur électrique configuré pour mesurer un paramètre lié à l'impédance électrique de la plaque vasculaire selon un mode de réalisation.
**Fig. 9**
   [Fig. 9] La figure 9 est une illustration schématique du dispositif de surveillance comprenant en outre un capteur d'ondes acoustiques configuré pour mesurer des ondes acoustiques provenant de la paroi artérielle et/ou de la plaque vasculaire.

### Description des modes de réalisation

La figure 1 illustre un mode de réalisation d'un système 1 de prédiction d'un risque de rupture ou décollement d'une plaque vasculaire pouvant entraîner un accident vasculaire cérébral par rupture de plaque. Le système 1 comprend :
- un dispositif de surveillance 2 (SURV) configuré pour être disposé à proximité de la carotide ou des troncs supra-aortiques d'un patient afin de mesurer les signaux représentatifs de la stabilité mécanique d'une ou plusieurs plaques vasculaires (d'athéromes ou d'autres origines) formées sur la paroi interne de la carotide,
- et une unité de calcul 10 (CALC) adaptée pour communiquer avec le dispositif de surveillance 2 et configurée pour analyser les signaux provenant du dispositif de surveillance 2 pour détecter s'il y a un risque de rupture d'au moins une portion de la plaque vasculaire. Typiquement, l'unité de calcul 10 peut être opérée par un praticien ou un groupe de praticien, et peut collecter et traiter les données d'un grand nombre de dispositifs de surveillance 2.

Le dispositif de surveillance 2 peut comprendre :
- un ou deux capteurs de vibrations 4 (SENS 1), notamment un ou plusieurs accéléromètre(s), configuré(s) pour mesurer des ondes mécaniques qui peuvent être générées par l'onde de pouls artériel traversant la plaque vasculaire et les parois sous-jacentes ,
- un capteur de température 5 (SENS 2) configuré pour mesurer la température de la plaque vasculaire,
- et un capteur de mouvement de plaque 6 (SENS 3), notamment un transducteur ultrasonore tel qu'un cristal piézoélectrique, configuré pour mesurer les mouvements de la plaque vasculaire.

Le dispositif de surveillance 2 comprend également une mémoire 8 (MEM) apte à stocker les signaux transmis par les capteurs 4, 5, 6 et une interface de communication 9 (INT 1). L'interface de communication 9 peut être par exemple :
- une interface radio courte portée notamment de type « Bluetooth », « Wifi » ou autre,
- ou encore une interface de communication en champ proche notamment de type NFC ou RFID.

Les signaux stockés dans la mémoire 8 peuvent être transmis à l'unité de calcul 10 via l'interface de communication 9.

L'unité de calcul 10 est configurée pour analyser les signaux notamment par une intelligence artificielle entraînée et détecter la survenue d'un AVC par rupture de plaque vasculaire. L'unité de calcul 10 comprend par exemple une unité centrale 12 (UC) dans laquelle est stocké le réseau neuronal entraîné. L'unité de calcul 10 comprend également une interface de communication 11 (INT 2) pour recevoir les signaux provenant du dispositif de surveillance 2. L'interface de communication 11 et l'unité centrale 12 peuvent être éventuellement distantes l'une de de l'autre. L'unité centrale 12 peut éventuellement être un serveur.

La fonction des capteurs 4, 5 et 6 est détaillée ci-dessous.

La rupture ou le décollement de la plaque vasculaire accrochée à la paroi interne de l'artère peut être provoqué par une onde mécanique générée par exemple par l'onde de pouls artériel. L'onde mécanique comprend une composante de cisaillement et une composante de compression, et peut être assimilée à une onde sismique. En mesurant les ondes mécaniques, le capteur de vibrations 4 est apte de mesurer un signal représentatif des propriétés biomécaniques de la plaque vasculaire et des parois sous-jacentes. Le capteur de vibrations 4 peut comprendre au moins un accéléromètre. Par exemple, le capteur de vibrations peut comprendre un accéléromètre 3 axes et un gyroscope 3 axes. Les données représentatives du signal mesuré par le capteur de vibrations 4 sont stockées dans la mémoire 8.

Le capteur de température 5 est apte de mesurer une variation locale de température représentative de la stabilité de la plaque. En effet, l'inflammation est une des principales caractéristiques d'une plaque instable. L'intensité du processus inflammatoire se traduit par une élévation de température dans la plaque. Selon un exemple de réalisation, le capteur de température 5 peut être configuré pour mesurer une onde thermique émise par la plaque dans le domaine des fréquences micro-ondes. Un tel capteur peut comprendre par exemple une antenne pour détecter lesdites ondes thermiques émises par la plaque vasculaire. Les données représentatives du signal mesuré par le capteur de température 5 sont stockées dans la mémoire 8.

Le capteur de mouvement de la plaque 6 est un capteur piézoélectrique configuré pour envoyer des ultrasons pour détecter en temps réel les mouvements de la plaque et définir s'ils sont réguliers ou irréguliers et anarchiques qui traduisent une arythmie ou un phénomène de perturbation de l'onde de pouls artériels. Les données représentatives du signal mesuré par le capteur de mouvement 6 sont stockées dans la mémoire 8.

En relation avec la figure 2, un système de prédiction selon un autre mode de réalisation est décrit. Il comprend en outre un appareil de communication mobile 13 communiquant avec le dispositif de surveillance 2 via une interface de communication de courte portée. L'appareil de communication mobile 13 comprend une application via laquelle le patient, porteur du dispositif de surveillance, peut récupérer les signaux stockés dans la mémoire 8. Le dispositif de surveillance transmet les signaux à l'appareil de communication mobile 13 via son interface de communication 9 qui est une interface de courte portée.

L'appareil de communication mobile 13 peut également être mis en communication avec l'unité de calcul 10. L'appareil de communication mobile peut par exemple transmettre les signaux récupérés via une interface de communication de type 2G, 3G, 4G ou 5G. L'unité de calcul 10 peut récupérer les signaux via son interface de communication 11 qui est une interface de longue portée.

L'appareil de communication mobile 13 peut être par exemple un téléphone portable intelligent (smart phone) ou une montre intelligente.

Le dispositif de surveillance 2 est un dispositif autonome. Le dispositif de surveillance 2 comprend une source d'énergie 7 (BATT) qui peut par exemple être rechargeable par un appareil externe, notamment par induction. La pile et les composants électroniques du dispositif d'acquisition de données peuvent être choisis de sorte que l'autonomie du dispositif soit au moins égale à un mois.

La figure 8 illustre une vue schématique d'un dispositif de surveillance 20 selon un autre mode de réalisation dans lequel il comprend en plus des trois autres capteurs 4, 5, 6 décrit ci-dessus, un quatrième capteur qui est un capteur électrique 24 (SENS 4).

Le capteur électrique 24 est configuré pour mesurer l'impédance électrique de la plaque vasculaire qui traduit un niveau de rigidité de la plaque qui a été identifié comme influent sur la stabilité de plaque vasculaire. La plaque vasculaire et le milieu environnant se comportent comme un milieu diélectrique. En mesurant l'impédance, il est possible d'extraire les paramètres diélectriques, à savoir la conductivité et la permittivité de ce milieu, représentatif du niveau de rigidité de la plaque. Selon un exemple de réalisation, le capteur d'impédance électrique est configuré pour appliquer un courant et mesurer une différence de potentiel. En connaissant la tension et le courant, on détermine la valeur de l'impédance de la plaque vasculaire à partir de la loi d'Ohm. La mesure en continu de l'impédance permet de suivre l'évolution de l'état de rigidité des plaques. Les données représentatives du signal mesuré par le capteur d'impédance 24 sont stockées dans la mémoire 8.

La figure 9 illustre une vue schématique d'un dispositif de surveillance 30 selon un autre mode de réalisation dans lequel il comprend en plus des quatre capteurs 4, 5, 6, 24 de la figure 8, un cinquième capteur passif d'ondes acoustiques 35 configuré pour mesurer les ondes acoustiques provenant de la paroi artérielle et de la plaque.

Il convient de noter que le système de prédiction peut également prédire un risque de rupture ou décollement d'une plaque vasculaire à partir d'un signal ou plusieurs signaux mesurés par les cinq capteurs 4, 5, 6, 24, 35 du dispositif de surveillance. Le dispositif de surveillance peut donc comprendre l'un quelconque ou plusieurs, ou une quelconque combinaison parmi les cinq capteurs 4, 5, 6, 24, 35 décrits ci-dessus pour mesurer un signal représentatif de la stabilité mécanique de la plaque vasculaire.

Selon un exemple de réalisation, le dispositif de surveillance comprend uniquement l'un des quatre capteurs parmi les cinq capteurs 4, 5, 6, 24, 35 décrits ci-dessus.

Selon un autre exemple de réalisation, le dispositif de surveillance peut comprendre une combinaison de deux capteurs parmi les cinq capteurs 4, 5, 6, 24, 35 décrits ci-dessus.

Selon encore un autre exemple de réalisation, le dispositif de surveillance peut comprend une combinaison de trois capteurs parmi les cinq capteurs 4, 5, 6, 24, 35 décrits ci-dessus.

Selon encore un exemple de réalisation, le dispositif de surveillance peut comprend une combinaison de quatre capteurs parmi les cinq capteurs 4, 5, 6, 24, 35 décrits ci-dessus.

De manière avantageuse, la fréquence de mesure des capteurs du dispositif de surveillance peut être réglée pour émettre et/ou recevoir des signaux provenant d'une région d'intérêt située à une certaine profondeur sous la peau.

La combinaison des capteurs varie en fonction des besoins cliniques.

Dans le cadre de l'utilisation du dispositif de surveillance pour suivre l'évolution dans le temps des propriétés biomécaniques d'une plaque vasculaire et/ou d'une paroi sous-jacente de la carotide, le dispositif de surveillance peut comprendre par exemple un capteur de vibrations 4 et/ou un capteur de température 5 et/ou un capteur de mouvements 6 et/ou un capteur électrique 24 et/ou un capteur de d'ondes acoustiques 35 configurés pour émettre et/ou recevoir des signaux provenant d'une plaque vasculaire et/ou d'une paroi sous-jacente de la carotide qui sont situées à une profondeur variable de la surface de la peau, généralement comprise entre 1-3 cm sous la peau. La fréquence de mesures du capteur de vibrations 4, du capteur de température 5, du capteur de mouvement 6, du capteur électrique 24 et du capteur d'ondes acoustiques 35 sont réglée pour émettre et/ou recevoir le signal provenant de la plaque vasculaire et/ou de la paroi sous-jacente de la carotide.

Dans le cadre de l'utilisation du dispositif de surveillance pour mesurer les modifications des propriétés biomécaniques de la veine jugulaire interne, le dispositif de surveillance peut comprendre par exemple un capteur de température 5 et/ou un capteur de mouvements 6 et/ou un capteur de d'ondes acoustiques 35 configurés pour émettre et/ou recevoir des signaux provenant d'une veine jugulaire interne qui est située à une profondeur variable de la surface de la peau selon l'anatomie du patient, généralement comprise entre 1 et 3 cm sous la peau. La fréquence de mesures du capteur de température 5, du capteur de mouvement 6 et du capteur d'ondes acoustiques 35 sont différentes et réglées pour émettre et/ou recevoir le signal provenant de la veine jugulaire.

La figure 3 illustre une vue schématique d'une carotide commune 100 qui se divise en deux au niveau du cou d'un patient : artère carotide interne 101 qui va irriguer le cerveau et l'artère carotide externe 102 qui va irriguer le cou et le visage. Le dispositif de surveillance 2 tel que décrit ci-dessus peut être placé en regard de la carotide interne juste après la bifurcation de la carotide commune de manière à mesurer en continu les différents signaux émis par une ou plusieurs plaques présentes sur la paroi interne de la carotide interne.

Sur la figure 3 est également illustrée de manière schématique une veine jugulaire interne 107 qui est une veine profonde du cou, au contact de l'artère carotide interne. Il existe une veine jugulaire interne de chaque côté du cou. La veine jugulaire interne chemine verticalement vers le bas, latéralement à l'artère carotide interne, puis à l'artère carotide commune.

De manière générale, le positionnement du dispositif peut être ajusté en fonction des besoins cliniques. Selon un autre mode de réalisation, il est également possible de positionner un deuxième dispositif de surveillance ou même plusieurs dispositifs pour surveiller non seulement la carotide interne mais également ses branches ou toute autre artère des troncs supra-aortiques. A titre d'exemple, il est possible de positionner un deuxième dispositif de surveillance juste avant la bifurcation de la carotide commune 100.

En référence à la figure 4, le dispositif de surveillance 2 se présenter sous la forme d'un patch 3 présentant une surface adhésive qui permet de coller le dispositif sur une zone de la surface extérieur de la peau 106 du cou, à proximité de la carotide 100.

En référence à la Figure 5, le dispositif de surveillance 2 peut également être un dispositif implantable sous cutané. Le dispositif de surveillance 2 comprend par exemple une enveloppe réalisée dans un matériau biocompatible. Le dispositif de surveillance est placé dans une sonde implantable. Il peut être placé sous la peau 106, à proximité de la carotide 100. Il est placé par le praticien qui réalise un décollement sous la peau. Le dispositif est mis en place en contact du muscle 104 sous la peau.

Un aspect de la présente divulgation est de permettre d'utiliser le système 1 pour surveiller en continu les modifications des propriétés biomécaniques d'une plaque interne et ou d'une paroi sous-jacente de la carotide. Dans le cadre de cette utilisation, le dispositif de surveillance peut comprendre un capteur de vibrations 4 et/ou un capteur de température.

Le procédé de surveillance peut être mis en oeuvre d'une manière non invasive en plaçant le dispositif de surveillance sur la peau d'un patient, à proximité de la carotide.

Le procédé de surveillance peut également être mise en oeuvre en plaçant le dispositif de surveillance sous la peau d'un patient, à proximité de la carotide.

En référence à la figure 6, un procédé mettant en oeuvre un système de prédiction d'un risque de rupture ou de décollement de plaque pouvant entraîner un AVC est décrit ci-dessous.

A l'étape E1, le capteur de vibrations 4 et/ou le capteur de température 5 et/ou le capteur de mouvements 6 et/ou le capteur électrique 24 et/ou le capteur de d'ondes acoustiques 35 sont réglés de manière à fonctionner dans une plage de fréquences adaptées pour émettre et/ou recevoir des signaux provenant d'une plaque vasculaire et/ou d'une paroi sous-jacente de la carotide.

A l'étape E1, le capteur de vibrations 4 et/ou le capteur de température 5 et/ou le capteur de mouvements 6 et/ou le capteur électrique 24 et/ou le capteur de d'ondes acoustiques 35 du dispositif de surveillance mesurent les signaux provenant de la plaque pendant une durée déterminée. La durée varie entre quelques heures à quelques semaines. Les capteurs sont par exemple préalablement programmés pour fonctionner pendant une durée déterminée. Cette durée est déterminée par le praticien en fonction des besoins cliniques.

Selon un exemple de réalisation, les trois capteurs 4, 5 et 6 du dispositif de surveillance mesurent les signaux provenant de la plaque et/ la paroi de la carotide pendant une durée déterminée.

Selon un autre exemple de réalisation, les quatre capteurs 4, 5, 6, 24 du dispositif de surveillance mesurent les signaux provenant de la plaque et/ou la paroi de la carotide pendant une durée déterminée.

Selon encore une autre réalisation, les cinq capteurs 4, 5, 6, 24, 35 du dispositif de surveillance mesurent les signaux provenant de la plaque et/ou la paroi de la carotide pendant une durée déterminée.

A l'étape E2, les signaux sont stockés dans la mémoire 8.

A l'étape E3, les signaux stockés dans la mémoire 8 du dispositif de surveillance sont envoyés vers l'unité de calcul 10 via l'interface de communication 9.

A l'étape E4, l'unité de calcul 10 analyse les signaux au moyen d'une intelligence artificielle entraînée pour détecter un risque de rupture ou de décollement de plaque pouvant entraîner un AVC. L'intelligence artificielle comprend un réseau neuronal entraîné à déterminer à partir des signaux collectés si la ou les plaques présentes dans la carotide présentent un risque de rupture.

Les étapes E3 et E4 sont par exemple effectuées chez le praticien. Il est ainsi possible pour le praticien d'obtenir une prédiction fiable sur le risque de la survenue d'un AVC par rupture de plaque chez le patient même si ce dernier ne présente aucun symptôme de la pathologie.

En variante, à l'étape E3, le patient peut également utiliser un appareil de communication mobile 13 pour communiquer avec le dispositif de surveillance périodiquement via une interface de communication de courte portée. L'appareil est par exemple un téléphone intelligent. Il comprend par exemple une application par laquelle le patient peut interroger le dispositif de surveillance pour recevoir les signaux stockés dans la mémoire 8 du dispositif de surveillance. L'appareil de communication 13 transmet ensuite les signaux via un réseau 4G ou 5 G vers l'unité de calcul 10. Ainsi, un diagnostic de l'évolution de l'état de stabilité des plaques peut être établi à distance par le praticien périodiquement, par exemple une fois par semaine.

Selon un mode de réalisation et en référence à la figure 7, lorsque l'intelligence artificielle est un réseau neuronal, le procédé peut comprendre en outre une étape d'apprentissage préliminaire pour entraîner l'intelligence artificielle de manière à déterminer à partir des signaux collectés si la ou les plaques présentes dans la carotide présentent un risque de rupture ou de décollement.

Plus précisément, l'étape d'apprentissage peut comprendre les sous-étapes suivantes.

A une sous-étape E01, une pluralité de dispositifs de surveillance est utilisée pour collecter des signaux chez une population dont le risque de rupture de la plaque vasculaire est connu. Les signaux, dits signaux de référence, sont stockés sur un serveur.

A une sous-étape E02, le réseau neuronal est entraîné avec les signaux de référence jusqu'à ce qu'il converge. Le réseau neuronal entrainé est ensuite stocké dans l'unité de calcul 10, notamment dans l'unité centrale 12.

Grâce à l'acquisition en continu d'un ensemble de signaux représentatifs de l'évolution de la stabilité de la plaque vasculaire et à l'utilisation d'une intelligence artificielle pour analyser les signaux obtenus, le système de la présente divulgation permet de prédire le risque de rupture de plaque pouvant entraîner la survenu d'un AVC. Le présent système peut être utilisé par le praticien, en complément des études morphologiques et histologiques, pour surveiller et évaluer localement l'évolution du risque de rupture des plaques et prévenir ainsi des conséquences cliniques graves telles que l'AVC. Le système de la présente divulgation permet ainsi d'identifier rapidement les individus qui ont besoin d'interventions, même en l'absence de symptômes pathologiques.

Un autre aspect de la présente divulgation est de permettre d'utiliser le système pour surveiller en continu les modifications des propriétés biomécaniques d'une veine jugulaire interne qui est située plus en profondeur sous la peau par rapport à la carotide. Dans le cadre de cette utilisation, le dispositif de surveillance peut comprend un capteur de température 5, un capteur de mouvements 6 et un capteur d'ondes acoustiques 35.

Le procédé de surveillance peut être mis en oeuvre d'une manière non invasive en plaçant le dispositif de surveillance sur la peau d'un patient, à proximité de la veine jugulaire interne.

Le procédé de surveillance peut également être mise en oeuvre en plaçant le dispositif de surveillance sous la peau d'un patient, à proximité de la veine jugulaire interne.

En référence à la figure 6, à l'étape E1, le capteur de température 5, le capteur de mouvements 6 et le capteur d'ondes acoustiques 35 du dispositif de surveillance 2 sont réglés de manière à fonctionner dans une plage de fréquences adaptées qui permettent d'émettre et/ou de recevoir des signaux provenant de la veine jugulaire interne pendant une durée déterminée.

La durée de la mesure varie entre quelques heures à quelques semaines. Les capteurs sont par exemple préalablement programmés pour fonctionner pendant une durée déterminée. Cette durée est déterminée par le praticien en fonction des besoins cliniques. Les signaux collectés sont représentatifs des modifications des propriétés biomécaniques de la veine jugulaire interne au cours du temps.

A l'étape E2, les signaux sont stockés dans la mémoire 8.

A l'étape E3, les signaux stockés dans la mémoire 8 du dispositif de surveillance sont envoyés vers l'unité de calcul 10 via l'interface de communication 9.

A l'étape E4, l'unité de calcul 10 analyse les signaux au moyen d'une intelligence artificielle entraînée pour détecter un risque de thrombose vasculaire. L'intelligence artificielle comprend un réseau neuronal entraîné à déterminer à partir des signaux collectés si les modifications des propriétés biomécaniques de la veine jugulaire interne présentent un risque de thrombose vasculaire.

Les étapes E3 et E4 sont par exemple effectuées chez le praticien. Il est ainsi possible pour le praticien d'obtenir une prédiction fiable sur le risque de la survenue d'une thrombose vasculaire chez le patient même si ce dernier ne présente aucun symptôme de la pathologie.

En variante, à l'étape E3, le patient peut également utiliser un appareil de communication mobile 13 pour communiquer avec le dispositif de surveillance périodiquement via une interface de communication de courte portée. L'appareil est par exemple un téléphone intelligent. Il comprend par exemple une application par laquelle le patient peut interroger le dispositif de surveillance pour recevoir les signaux stockés dans la mémoire 8 du dispositif de surveillance. L'appareil de communication 13 transmet ensuite les signaux via un réseau 4G ou 5 G vers l'unité de calcul 10. Ainsi, il est possible de suivre l'évolution des propriétés biomécaniques de la veine jugulaire interne à distance par le praticien périodiquement, par exemple une fois par semaine.

Selon un mode de réalisation et en référence à la figure 7, lorsque l'intelligence artificielle est un réseau neuronal, le procédé peut comprendre en outre une étape d'apprentissage préliminaire pour entraîner l'intelligence artificielle de manière à déterminer à partir des signaux collectés au cours du temps de caractériser un risque de survenue d'une thrombose vasculaire.

Plus précisément, l'étape d'apprentissage peut comprendre les sous-étapes suivantes.

A une sous-étape E01, une pluralité de dispositifs de surveillance est utilisée pour collecter des signaux chez une population dont le risque de thrombose vasculaire est connu. Les signaux, dits signaux de référence, sont stockés sur un serveur.

A une sous-étape E02, le réseau neuronal est entraîné avec les signaux de référence jusqu'à ce qu'il converge. Le réseau neuronal entrainé est ensuite stocké dans l'unité de calcul 10, notamment dans l'unité centrale 12.

Grâce à l'acquisition en continu d'un ensemble de signaux représentatifs de l'évolution des modifications des propriétés biomécaniques de la veine jugulaire interne et à l'utilisation d'une intelligence artificielle pour analyser les signaux obtenus, le système de la présente divulgation permet également de prédire le risque de formation d'un caillot de sang dans la veine jugulaire interne du cou, pouvant entraîner la survenu d'une thrombose vasculaire. Le procédé décrit ci-dessous permet d'aider le praticien, en complément des examens connus, par exemple un écho-Doppler qui permet de visualiser le caillot, à évaluer le risque de thrombose jugulaire, afin de pouvoir prendre en charge le patient rapidement.

En outre, l'utilisation du système n'est pas limitée uniquement à prédire l'AVC. Il peut être mis en place chez un patient après l'occurrence d'un AVC pour prédire le risque de récidive après un traitement des plaques vasculaires.

Bien que le système de la présente invention présente des avantages particuliers pour la prédiction de la survenue d'un AVC, le système peut s'appliquer de manière générale pour détecter précocement les patients à risque de développer des maladies cardiovasculaires. Le système peut également s'appliquer lors d'un essai clinique pour évaluer l'efficacité d'un nouveau traitement des plaques vasculaire.

## Revendications

1. Système de prédiction (1) d'une rupture au moins partielle ou un décollement de plaque vasculaire pouvant entraîner un accident vasculaire cérébral, ladite plaque vasculaire étant présente sur une paroi artérielle choisie parmi une paroi de carotide et une paroi de tronc supra-aortique, ledit système comprenant :
- un dispositif de surveillance (2) apte à être placé à proximité de la plaque vasculaire, ledit dispositif comprenant au moins un capteur de température (5) configuré pour mesurer la température de la plaque vasculaire, au moins un capteur de vibrations (4) configuré pour mesurer des ondes mécaniques propagées dans ladite paroi artérielle, au moins un capteur de mouvement de la plaque vasculaire (6) configuré pour mesurer des mouvements de la plaque vasculaire, une mémoire (8) apte à stocker des signaux transmis par lesdits capteurs, une interface de communication (9), une source d'énergie (7) configurée pour alimenter lesdits capteurs et l'interface de communication (9),
- une unité de calcul (10) adaptée pour communiquer avec le dispositif de surveillance (2) et configurée pour analyser des mesures du capteur de vibrations (4), du capteur de température (5) et du capteur de mouvement de plaque vasculaire (6) provenant du dispositif de surveillance (2) par une intelligence artificielle entraînée pour détecter s'il y a un risque de rupture au moins partielle ou décollement de la plaque vasculaire.

2. Système selon la revendication 1, dans lequel le dispositif de surveillance (20) comprend en outre au moins un capteur électrique (24) configuré pour mesurer un paramètre lié à l'impédance électrique de la plaque vasculaire.

3. Système selon la revendication 1 ou 2, dans lequel le dispositif de surveillance (30) comprend en outre au moins un capteur d'ondes acoustiques (35) pour mesurer les ondes acoustiques provenant de la paroi artérielle et/ou de la plaque vasculaire.

4. Système selon l'une des revendications 1 à 3, dans lequel le dispositif de surveillance (2, 20, 30) se présente sous la forme d'un patch apte à être collé sur une surface externe de la peau (106) d'un patient.

5. Système selon l'une des revendications à 1 à 3, dans lequel le dispositif de surveillance (2, 20, 30) se présente sous la forme d'un implant sous-cutanée apte à être inséré sous la peau d'un patient.

6. Système selon l'une des revendications 1 à 5, dans lequel le capteur de température (5) est un capteur d'ondes thermique émises par la plaque vasculaire.

7. Système selon l'une des revendications 1 à 6, dans lequel le capteur de vibrations (4) comprend un accéléromètre.

8. Système selon la revendication 7, dans lequel le capteur de vibrations (4) comprend un accéléromètre 3 axes et un gyroscope 3 axes.

9. Système selon l'une des revendications 1 à 8, dans lequel le capteur de mouvement de la plaque vasculaire (6) est une sonde ultrasonore.

10. Système selon l'une des revendications 1 à 9, dans lequel la source d'énergie (7) est une pile rechargeable par induction.

11. Système selon l'une des revendications 1 à 10, dans lequel l'interface de communication (9) est choisie parmi une interface radio courte portée ou une interface de communication en champ proche.

12. Système selon l'une des revendications 1 à 11, comprenant en outre au moins un appareil mobile de communication (13) adapté pour communiquer à distance avec le dispositif de surveillance (2) via ladite interface de communication (9) qui est une interface de courte portée et pour transmettre des signaux vers l'unité de calcul (10) via une interface de communication de longue portée (11) appartenant à l'unité de calcul (10).

13. Système selon la revendication 3, dans lequel le dispositif de surveillance comprend au moins un capteur de température (5) et/ou au moins un capteur de mouvement (6) et/ou au moins un capteur d'ondes acoustiques (35), la fréquence de mesure desdits capteurs étant réglée pour émettre et/ou recevoir un signal provenant d'une veine jugulaire interne.

## Patentansprüche

1. System (1) zur Vorhersage einer wenigstens teilweisen Ruptur oder einer Ablösung einer Gefäßplaque, die zu einem Schlaganfall führen kann, wobei die Gefäßplaque an einer Arterienwand vorhanden ist, die aus einer Karotiswand und einer Wand des supraaortalen Stammes gewählt ist, wobei das System umfasst:
- eine Überwachungsvorrichtung (2), die in der Nähe der Gefäßplaque angebracht werden kann, wobei die Vorrichtung wenigstens einen Temperatursensor (5) umfasst, der dazu ausgebildet ist, die Temperatur der Gefäßplaque zu messen, wenigstens einen Vibrationssensor (4), der dazu ausgebildet ist, mechanische Wellen zu messen, die sich in der Arterienwand ausbreiten, wenigstens einen Bewegungssensor für die Gefäßplaque (6), der dazu ausgebildet ist, Bewegungen der Gefäßplaque zu messen, einen Speicher (8), der dazu ausgebildet ist, von den Sensoren übermittelte Signale zu speichern, eine Kommunikationsschnittstelle (9), eine Energiequelle (7), die dazu ausgebildet ist, die Sensoren und die Kommunikationsschnittstelle (9) mit Energie zu versorgen,
- eine Recheneinheit (10), die dazu geeignet ist, mit der Überwachungsvorrichtung (2) zu kommunizieren und dazu ausgebildet ist, Messungen des Vibrationssensors (4), des Temperatursensors (5) und des Gefäßplaquebewegungssensors (6) zu analysieren, die von der Überwachungsvorrichtung (2) stammen, mittels einer künstlichen Intelligenz, die darauf trainiert ist, zu erkennen, ob die Gefahr einer wenigstens teilweisen Ruptur oder Ablösung der Gefäßplaque besteht.

2. System nach Anspruch 1, wobei die Überwachungsvorrichtung (20) ferner wenigstens einen elektrischen Sensor (24) umfasst, der dazu ausgebildet ist, einen Parameter zu messen, der mit der elektrischen Impedanz der Gefäßplaque zusammenhängt.

3. System nach Anspruch 1 oder 2, wobei die Überwachungsvorrichtung (30) ferner wenigstens einen Schallwellensensor (35) umfasst, um Schallwellen zu messen, die von der Arterienwand und/oder der Gefäßplaque ausgehen.

4. System nach einem der Ansprüche 1 bis 3, wobei die Überwachungsvorrichtung (2, 20, 30) in Form eines Patch vorliegt, das auf eine Außenfläche der Haut (106) eines Patienten geklebt werden kann.

5. System nach einem der Ansprüche 1 bis 3, wobei die Überwachungsvorrichtung (2, 20, 30) in Form eines subkutanen Implantats vorliegt, das unter die Haut eines Patienten eingesetzt werden kann.

6. System nach einem der Ansprüche 1 bis 5, wobei der Temperatursensor (5) ein Sensor für von der Gefäßplaque abgegebene Wärmewellen ist.

7. System nach einem der Ansprüche 1 bis 6, wobei der Vibrationssensor (4) einen Beschleunigungsmesser umfasst.

8. System nach Anspruch 7, wobei der Vibrationssensor (4) einen 3-Achsen-Beschleunigungsmesser und ein 3-Achsen-Gyroskop umfasst.

9. System nach einem der Ansprüche 1 bis 8, wobei der Bewegungssensor der Gefäßplaque (6) eine Ultraschallsonde ist.

10. System nach einem der Ansprüche 1 bis 9, wobei die Energiequelle (7) eine induktiv aufladbare Batterie ist.

11. System nach einem der Ansprüche 1 bis 10, wobei die Kommunikationsschnittstelle (9) aus einer Kurzstrecken-Funkschnittstelle oder einer Nahfeld-Kommunikationsschnittstelle gewählt ist.

12. System nach einem der Ansprüche 1 bis 11, das ferner wenigstens ein mobiles Kommunikationsgerät (13) umfasst, das dazu geeignet ist, über die Kommunikationsschnittstelle (9), bei der es sich um eine Kurzstreckenschnittstelle handelt, aus der Ferne mit der Überwachungsvorrichtung (2) zu kommunizieren und über eine zur Recheneinheit (10) gehörende Langstreckenkommunikationsschnittstelle (11) Signale an die Recheneinheit (10) zu übertragen.

13. System nach Anspruch 3, wobei die Überwachungsvorrichtung wenigstens einen Temperatursensor (5) und/oder wenigstens einen Bewegungssensor (6) und/oder wenigstens einen Schallwellensensor (35) umfasst, wobei die Messfrequenz der Sensoren dazu eingestellt ist, ein Signal aus einer inneren Halsvene zu senden und/oder zu empfangen.

## Claims

1. A system (1) for predicting an at least partial rupture or the detachment of vascular plaque that could lead to a stroke, with said vascular plaque being present on an arterial wall selected from among a carotid wall and a supra-aortic trunk wall, said system comprising:
- a monitoring device (2) able to be placed in the vicinity of the vascular plaque, said device comprising at least one temperature sensor (5) configured to measure the temperature of the vascular plaque, at least one vibration sensor (4) configured to measure mechanical waves propagated in said arterial wall, at least one motion sensor (6) for the vascular plaque configured to measure movements of the vascular plaque, a memory (8) able to store signals transmitted by said sensors, a communication interface (9), a power source (7) configured to power said sensors and the communication interface (9);
- a computation unit (10) adapted to communicate with the monitoring device (2) and configured to analyze measurements of the vibration sensor (4), of the temperature sensor (5) and of the vascular plaque motion sensor (6) originating from the monitoring device (2) by artificial intelligence trained to detect whether there is a risk of at least partial rupturing or detachment of the vascular plaque.

2. The system as claimed in claim 1, wherein the monitoring device (20) further comprises at least one electrical sensor (24) configured to measure a parameter related to the electrical impedance of the vascular plaque.

3. The system as claimed in claim 1 or 2, wherein the monitoring device (30) further comprises at least one acoustic wave sensor (35) for measuring the acoustic waves originating from the arterial wall and/or from the vascular plaque.

4. The system as claimed in any of claims 1 to 3, wherein the monitoring device (2, 20, 30) is in the form of a patch able to be adhered to an external surface of the skin (106) of a patient.

5. The system as claimed in any of claims 1 to 3, wherein the monitoring device (2, 20, 30) is in the form of a subcutaneous implant able to be inserted under the skin of a patient.

6. The system as claimed in any of claims 1 to 5, wherein the temperature sensor (5) is a sensor for detecting thermal waves emitted by the vascular plaque.

7. The system as claimed in any of claims 1 to 6, wherein the vibration sensor (4) comprises an accelerometer.

8. The system as claimed in claim 7, wherein the vibration sensor (4) comprises a 3-axis accelerometer and a 3-axis gyroscope.

9. The system as claimed in any of claims 1 to 8, wherein the vascular plaque motion sensor (6) is an ultrasound probe.

10. The system as claimed in any of claims 1 to 9, wherein the power source (7) is an induction-rechargeable battery.

11. The system as claimed in any of claims 1 to 10, wherein the communication interface (9) is selected from among a short-range radio interface or a near-field communication interface.

12. The system as claimed in any of claims 1 to 11, further comprising at least one mobile communication appliance (13) adapted to remotely communicate with the monitoring device (2) via said communication interface (9), which is a short-range interface, and to transmit signals to the computation unit (10) via a long-range communication interface (11) forming part of the computation unit (10).

13. The system as claimed in claim 3, wherein the monitoring device comprises at least one temperature sensor (5) and/or at least one motion sensor (6) and/or at least one acoustic wave sensor (35), with the measurement frequency of said sensors being set so as to transmit and/or receive a signal originating from an internal jugular vein.
